# EUROPEAN PATENT APPLICATION

(11) **EP 0 602 891 A1**
(43) Date of publication of application: **22.06.1994**
(21) Application number: 93309869.1
(22) Date of filing: 08.12.1993
(51) Int. Cl.: A01N 65/00, A01N 37/40, A01N 31/16, C02F 1/50

(54) **Legionella effective biocide for aqueous based systems**

(30) Priority: 08.12.1992 GB 9225625
(71) Applicant: W.R. Grace & Co.-Conn., New York, New York 10036 (US)
(72) Inventor: Rimmer, Karen, Runcorn, Cheshire (GB); Little, Gavin, Cheshire (GB); Clarkson, Douglas, Lancashire (GB); Bennison, John James, Cheshire (GB); Hamels, Walter, 6900 Heidelberg (DE)
(74) Representative: Bentham, Stephen

(57) **Abstract**

A method of controlling Gram-negative bacteria of the family *Legionellaceae* in an aqueous system which comprises adding to the system a citrus fruit seed extract, preferably grapefruit seed extract, is useful in industrial water systems, such as cooling water systems and in the paper-making industry.

## Description

This invention relates to the treatment of aqueous systems, especially industrial water systems such as cooling water systems and water systems used in paper pulping and manufacture.

In industrial cooling water systems, for instance in industrial cooling towers, the water used is not, of course, sterile with the result that bacteria accumulate in the system and this quite commonly gives rise to a slimy deposit on the surfaces of the system which come into direct contact with the cooling water. A similar situation applies in paper making; slime can deposit on any of the surfaces with which the water comes into contact including the paper pulping bath, on the paper web and in the recirculating back pipe. Again such problems arise in the extraction and refining of sugar.

A large variety of different microbiological control agents have been used for the purpose of killing these bacteria and/or inhibiting slime formation or for dispersing and killing microbiological slime. These chemicals are principally biostats such as lime or sulphur dioxide or, more generally, biocides, for example isothiazolones, methylene bis (thiocyanate), quaternary ammonium compounds and chlorine release agents.

Particular problems are associated with *Legionella* species in view of the dangers associated with these bacteria.

Citrus fruit seed extract, and in particular grapefruit seed extract, has been disclosed as having anti-bacterial, anti-algae and anti-fungal properties and has been used in the treatment of domestic water and pools, spas and ponds as a disinfectant or to control algae. It has also been used as a preservative on fruit and vegetables and in cosmetics.

It has been suggested that this extract comprises various naturally occurring salts of ascorbic acid, as well as other naturally occurring compounds, including fruit sugars, the amino acid glycine and vitamin E. The extract comprises "essential oils" some of which have antimicrobial activity, (see EP-A-350 275). Analyses of the chemical constituents of citrus fruit seed extracts have also been published: see for example, Abdel-Rahaman, Grasas Y Aceites, Vol. 31, pp 331-333 (1980) and Habib et. al., JAOCS, Vol. 63, pp 1192-1197 (1986). None of these clearly identify the active antimicrobial species in citrus fruit seed extract.

However, there has to the applicant's knowledge, been no disclosure of the use of citrus fruit extract in industrial water systems. Such system, and in particular paper-making systems, are generally subject to a much higher level of contamination and impurity than domestic water systems.

Conventionally, biocides used in such systems include chlorine and ozone. Such biocides are not naturally occurring materials and are considerably less environmentally friendly than citrus fruit seed extract. Their use in the treatment of industrial water systems does not suggest that natural products such as citrus fruit extract be used for the control of bacteria and in particular *Legionellaceae,* in industrial water systems which are likely to be subject to high levels of contamination.

It has surprisingly been found according to the present invention that citrus fruit seed extract is effective for killing the Gram negative bacterial family *Legionellaceae,* particularly *Legionella pneumophila.* The ability of citrus fruit seed extract to control *Legionellaceae* would not be expected in view of its known uses. The extract, in view of its generally less aggressive properties in use possesses considerable advantages over the use of conventional biocides in industrial water systems, particularly in the level of care required in handling the extract.

Accordingly, the present invention provides a method of controlling Gram-negative bacteria of the family *Legionellaceae* in an aqueous system which comprises adding to the system a citrus fruit seed extract in an amount effective to control or inhibit the growth of the bacteria.

The invention also provides a composition, suitable for addition to an industrial aqueous system, which comprises citrus fruit seed extract, and an industrial water treatment additive which is a dispersing agent, papermaking additive or cooling water additive.

The invention further provides a pack for simultaneous, sequential or separate addition to a paper-making or cooling water system which comprises:-
(a) a composition comprising citrus fruit seed extract, and
(b) a dispersing agent, a paper-making additive or cooling water additive.

The present invention is particularly useful in relation to the treatment of industrial cooling water systems and in the paper-making industry in order to prevent the growth of bacteria of the family *Legionellaceae.* The invention may also be used in other industrial water systems.

In the present invention, various types of citrus fruit seed extract may be used, such as grapefruit, orange or lemon seeds.

Preferably grapefruit seed extract is used.

It has been suggested that the active antibacterial substances in grapefruit seed extract are 2,4,4'-trichloro-2-hydroxydiphenyl ether and methyl-p-hydroxybenzoate. (N. Atsuyoshi et al, Chemical Abstract, 1992, Vol 116, 80284X). In view of this, the applicants consider it likely that, at least in part, the activity of citrus fruit seed extract in the control of *Legionellaceae* arises from one or other of these compounds or from the combination of them. Accordingly, a feature of the present invention is the control of *Legionellaceae* by these compounds, either singly or in combination.

The citrus fruit seed extract used in the present invention may be obtained in known conventional manner. It may for example be obtained by extraction from crushed seeds using a solvent such as acetone, an alcohol, glycerine or propylene glycol. Alternatively, the extract may be obtained as a powder by freeze-drying crushed citrus fruit seeds. Grapefruit seed extract is commercially available and the product Citricidal, available from Biochem research, Germany, is one example of an extract suitable for use in the invention.

The citrus fruit seed extract will, in accordance with the invention, generally be used in combination with one or more carriers or diluents, and preferably such components will therefore be present in the composition of the present invention. Particularly where the extract is itself in liquid form, it is possible however to use the grapefruit seed extract without any additional carrier or diluent.

Preferably the composition of the invention is in the form of a liquid, and more preferably comprises as carrier or diluent one or more solvents, in which the citrus fruit seed extract is soluble. Alternatively, however the composition may be solid, for example in the form of a powder, optionally comprising a carrier in addition to dried citrus fruit seed extract.

The solvents used in the liquid composition are preferably hydrophilic organic solvents which are miscible with water and can give storable, stable compositions. Examples of suitable hydrophilic solvents include glycerine, glycols, such as ethylene glycol, propylene glycol, diethylene glycol, dipropylene glycol and butyl diglycol; glycol ethers, such as 2-methoxyethanol, 2-ethoxyethanol, 2-phenoxyethanol, diethylene glycol monomethyl ether, propylene glycol monomethyl ether, dipropylene glycol monomethyl ether and tripropylene glycol monomethyl ether; and alcohols containing up to 8 carbon atoms. Mixtures of two or more solvents may also be used. Glycerine and glycols are particularly preferred.

Optionally, the liquid composition of the invention may further comprise one or more inert solid carriers which may for example be used to adjust the flowabilty of the composition. A suitable carrier is silicon dioxide. Such carriers may alternatively be used in the absence of solvent where the composition is solid.

Preferably the composition of the invention comprises from 40 to 60%, for example about 50% by weight of citrus fruit seed extract. The balance of the composition may consist of solvent, or a mixture of solvent and solid carrier (typically up to about 30% by weight of the composition may be solid carrier) or additionally the composition may comprise one or more dispersing agents.

The composition may also contain one or more dispersing agents. Examples of suitable dispersing agents include cationic, anionic, non-ionic or amphoteric surfactants; non-ionic surfactants are preferred.

Typical surfactants which can be used include ethylene oxide adducts, especially ethoxylated phenols having the general formula: 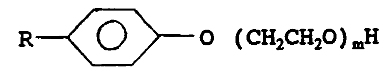
where m represents 2 to 40 and R represents CₙH₂ₙ₊₁ in which n is from O to 18, as well as alkylamine polyoxypropylenepolyoxyethylene adducts and alkylolamides.

Preferred ethoxylates are those derived from phenol itself, nonyl phenol and dodecyl phenol and those containing 4 to 15 ethoxylate groupings. Especially preferred is "Ethylan HB4" which is a phenol ethoxylate containing about 4 ethoxylate units.

Typical alkylamine polyoxypropylene polyoxyethylene adducts include N,N,N',N'-polyoxyethyelene-polyoxypropylene-ethylenediamine block copolymers, for example those having the formula:

[H(C₂H₄O)ₓ(C₃H₆O)_{y}]-NC₂H₄N-[(C₃H₆O)_{y}(C₂H₄O)ₓ]₂

in which each x and y has a value which can differ from one block to the next. These materials are available commercially as "Tetronics", varying in molecular weight and the relative proportions of ethylene oxide and propylene oxide units; in general the ethylene oxide units represent 10 to 80% by weight of the product while propylene units provide a molecular weight of, say, 2,000 to 25,000.

Typical alkylolamides which can be used include those obtained from a fatty acid containing, say, 8 to 18 carbon atoms, for example coconut fatty acids, and an alkanolamine, preferably ethanolamine or diethanolamine. Some such materials are available commercially under the trade marks Concenstate.

Typically the ratio of dispersant to biocide will be from 1:20 to 200:1 preferably from 1:5 to 10:1.

The present invention finds utility in different aqueous systems but, more particularly, in the paper-making industry and in cooling water systems.

In paper making, the citrus fruit seed extract may be added to the paper pulping bath, the recirculating backwater, or, for example, to a holding or storage tank containing generally moist, pulp or containing starch, calcium carbonate or paper coating masses. The extract may be added together or separately with one or more chemical additives used in paper making. Such additives include starch, for example potato or corn starch, titanium dioxide, a defoamer such as fatty acid alcohol, a size for example a rosin size based on abietic acid, a neutral size based on alkyl ketene dimer or a succinic acid anhydride based size, a wet strength resin such as, if neutral, an epichlorohydrin polyamide or, if acid, a melamine- or urea-formaldehyde resin, various polymers used as dispersants or retention aids such as polyacrylates, polymethacrylates, polyamides and polyacrylamides, clay, chalk, fillers such as carboxymethyl cellulose, polyvinyl alcohol and optical brightening agents.

In cooling water systems, the citrus fruit seed extract may be introduced at any location where they will be quickly and efficiently mixed with the water of the system although it will generally be most convenient to add them to the make-up or feed water lines through which the water enters the system. Typically, an injector calibrated to deliver a pre-determined amount periodically or continuously to the make-up water is employed. Conventional water treatment chemical additives such as scale or corrosion inhibitors and/or dispersants can also be added with the extract.

Typically, the amount of composition of the present invention added to the aqueous system will be such as to provide a concentration of citrus fruit seed extract from 0.5 to 500 ppm, preferably 25 to 200 ppm, for example about 50ppm. The concentration of citrus fruit seed extract will range depending upon the nature and concentration of the bacteria present but an amount effective to control the bacteria present should be used.

In accordance with the invention, citrus fruit seed extract may be used alone or in combination with other biocides. In particular effective control of bacteria may be achieved by alternately adding to an aqueous system different biocides at regular intervals.

The present invention will now be illustrated with reference to the following Example.

### Example

Evaluation of Grapefruit seed extract (GSE) against *Legionella pneumophila*

This was carried out, using the grapefruit seed extract Citricidal, available commercially from Bio-Chem Research, Germany. The product comprised 50% by weight grapefruit seed extract.

Dilutions of Citricidal were prepared and a bacterial suspension *(Legionella pneumophila)* was added together with a known level of organic soiling. As a control, samples were prepared containing bacterial suspension and organic soiling but no Citricidal. The samples were then incubated for contact times of 6 or 24 hours. Neutralising medium was then added to the test mixture to prevent further activity of the Citricidal.

The number of surviving organisms was then quantified by plating out aliquots of the neutralised mixture. Culture plates were incubated for 7 days and the bacterial counts were recorded.

The results in tables 1 and 2 from two separate screenings were expressed as surviving organisms/ml.

**Table 1**

| (Screening 1) Effect of Citricidal against *Legionella pneumophila* | | |
|---|---|---|
| GSE Concentration | INCUBATION PERIOD 5 Days | |
| | CONTACT TIMES | |
| ppm | 6 hour | 24 hour |
| 25 | 6.0 x 10³ | 5.0 x 10³ |
| 50 | NIL | NIL |
| 100 | NIL | NIL |
| 200 | NIL | NIL |
| Control | 3.7 x 10⁷ | 3.8 x 10⁷ |

**Table 2**

| (Screening 2) Effect of Citricidal against *Legionella pneumophila* | | |
|---|---|---|
| GSE Concentration | INCUBATION PERIOD 5 Days | |
| | CONTACT TIMES | |
| ppm | 6 hour | 24 hour |
| 25 | 2.55 x 10⁴ | 1.75 x 10⁴ |
| 50 | 2.0 x 10³ | NIL |
| 100 | NIL | NIL |
| 200 | NIL | NIL |
| Control | 8.3 x 10⁷ | 7.85 x 10⁷ |

In screening No 1 a log 4 reduction in recoverable organisms was achieved at a concentration of 25ppm of grapefruit seed extract following a 6 hour or 24 hour contact.

In screening No 2 a 25ppm concentration achieved a log 3 reduction for corresponding contact times. This was probably due to a higher bacterial load (indicated by control counts).

For both screenings at a concentration of greater than or equal to 50ppm of grapefruit seed extract, a greater than log 4 reduction in recoverable organisms was achieved for a contact time of either 6 hours or 24 hours.

## Claims

1. A method of controlling Gram-negative bacteria of the family *Legionellaceae* in an aqueous system which comprises adding to the system a citrus fruit seed extract in an amount effective to control or inhibit the growth of the bacteria.

2. A method according to claim 1 which comprises adding to the system grapefruit seed extract.

3. A method according to claim 1 or 2 of controlling *Legionellaceae* in an industrial water system.

4. A method according to claim 3 of controlling *Legionellaceae* in an industrial cooling water system or a paper-making system.

5. A method according to any one of the preceding claims in which the total amount of citrus fruit seed extract is from 0.5 to 500 ppm.

6. A method according to claim 5 in which the total amount of citrus fruit seed extract is about 50ppm.

7. A composition suitable for addition to an industrial aqueous system, which comprises citrus fruit seed extract, and an industrial water treatment additive which is a dispersing agent, papermaking additive or cooling water additive.

8. A composition according to claim 7 which is a liquid and further comprises a hydrophilic organic solvent in which the citrus fruit seed extract is soluble and optionally a solid carrier.

9. A composition according to claim 8 which comprises, as solvent, glycerine and optionally, as solid carrier, silicon dioxide.

10. A composition according to claim 8 or 9 which comprises by weight from 40 to 60% of citrus fruit seed extract, from 60 to 10% of solvent and, optionally, up to 30% of a solid carrier.

11. A composition according to any one of claims 7 to 10 which comprises a dispersing agent.

12. A composition according to claim 11 which comprises as dispersing agent, an ethylene oxide adduct, alkylamine polyoxypropylene polyoxyethylene adduct or alkylolamide.

13. A composition according to any one of claims 7 to 12 which comprises as paper-making additive a defoamer or as cooling water additive, a scale or corrosion inhibitor.

14. A pack for simultaneous, sequential or separate addition to a paper-making or cooling water system which comprises:-
(a) a composition comprising citrus fruit seed extract, and
(b) a dispersing agent, a paper-making additive or cooling water additive.

15. A pack according to claim 14 which comprises as paper-making additive a defoamer or as cooling water additive, a scale or corrosion inhibitor or a dispersing agent.

16. A method according to any one of claims 1 to 6 which comprises adding to the system a composition as claimed in any one of claims 7 to 13, or a pack as claimed in claim 14 or 15.
